# EUROPEAN PATENT SPECIFICATION

(11) **EP 1 562 661 B1**
(45) Date of publication and mention of the grant of the patent: **24.06.2009**
(21) Application number: 02793322.5
(22) Date of filing: 18.11.2002
(51) Int. Cl.: A61M 5/32, A61M 5/50

(54) **GUARD MECHANISM ATTACHABLE TO STANDARD SYRINGE TO TRANSFORM IT INTO A SAFETY SYRINGE**
SCHUTZVORRICHTUNG BEFESTIGBAR AN EINER STANDARDSPRITZE UM SIE IN EINE SICHERHEITSSPRITZE UMZUWANDELN
MECANISME DE PROTECTION POUVANT ETRE FIXE A UNE SERINGUE CLASSIQUE POUR TRANSFORMER CELLE-CI EN SERINGUE DE SURETE

(43) Date of publication of application: 17.08.2005
(73) Proprietor: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo
(86) International application number: PCT/IT2002/000730
(87) International publication number: WO 2004/045685

(56) References cited:
- WO-A-01/85239
- WO-A-99/37345
- US-A- 5 267 976
- US-A- 5 273 541
- US-A- 5 376 080
- US-A- 5 433 712
- US-A- 5 843 041
- US-B1- 6 206 853

## Description

The present invention refers to a guard mechanism attachable to a conventional syringe to make it into a disposable automatic safety syringe. The present invention also refers to a disposable automatic safety syringe obtained with said guard mechanism.

As is known, a syringe generally comprises a cylindrical body open at the rear to receive a plunger. A needle hollow on the inside is mounted at the head of the syringe body. On retraction of the plunger the liquid contained in a vial is drawn into the syringe body through the needle. On pressing on the plunger the liquid contained in the syringe body is injected, through the needle, into the patient's body.

Because of health regulations and to avoid transmission of infectious diseases, syringes must generally be used only once and then discarded. For this reason, there is a growing market demand for disposable syringes able to prevent further use.

Moreover, syringes generally present drawbacks from the point of view of safety. In fact, once the syringe has been used, the needle remains exposed at the head of the syringe body, with the risk of injuries and accidental needle sticks.

Patent application PCT WO 99/37345 describes a disposable safety syringe which has a needle covering sleeve mounted axially on the body of the syringe and slidable from a retracted position, wherein it leaves the needle exposed to allow injection, to a forward position wherein it completely covers the needle, preventing re-use of the syringe and acting as a guard against accidental needle sticks.

Once the injection has been performed, the sleeve is automatically brought into the extracted safety position, by means of an automatic mechanism and without any intervention by the user. However, this solution presents a certain complexity due to the presence of various additional elements for operation of the automatic mechanism.

US 5,376,080 discloses a retractable needle syringe comprising an outer container within which a needle holder body together with a needle can move axially.

WO 01/85239 discloses a guard mechanism according to the preamble of claim 1.

The object of the present invention is to eliminate the drawbacks of the prior art, providing a guard mechanism that is extremely versatile and suitable to be applied to a conventional syringe to make it into a disposable automatic safety syringe.

Another object of the present invention is to provide such a guard mechanism that is economical, simple to make and simple to assemble.

Another object of the invention is to provide such a mechanism that allows a controlled intervention of the guard on the syringe on completion of the injection.

Yet another object of the present invention is to provide such a disposable automatic safety syringe that is able to prevent both further attempts at use of the needle and accidental injury after use thereof.

These objects are achieved in accordance with the invention with the guard mechanism according to appended independent claim 1.

Advantageous embodiments of the invention are apparent from the dependent claims.

According to the invention a guard mechanism is applied to a standard syringe to make it into a disposable automatic safety syringe.

The syringe comprises a syringe body hollow on the inside and open at the front and the rear, a plunger that can slide inside the syringe body with an injection stroke extending from a retracted syringe-filling position to a forward syringe-emptying position, and an injection needle supported by a needle-carrying support engageable to the fore end of the syringe body. The plunger is provided at the rear with a shaft that can be operated manually and brought out of the syringe body through the rear end thereof.

The guard mechanism comprises a sleeve, spring means and an abutment member for the spring.

The abutment member for the spring is suitable to be mounted at the head of the syringe body and can act as a support for a injection needle.

The sleeve may be mounted so that it can slide over the spring abutment member and the syringe body, to pass from a retracted position of use of the syringe to a forward position of safety, wherein it covers the needle.

The spring means may be disposed under compression in the front part of the sleeve between the sleeve and the abutment member to urge the axial movement of the sleeve in the forward position of safety with respect to the syringe body.

The sleeve may be locked in the position of use of the syringe by means of locking means, in reciprocal engagement, provided in the rear part of the sleeve and of the syringe body.

In the rear part of the shaft, on the other hand, operating means may be provided to release the locking means when the plunger reaches the end of the injection stroke, so as to allow the action of the spring means which automatically generate the axial movement of the sleeve with respect to the syringe body. Such operating means can be made integral with the shaft, or they can form part of the guard mechanism and thus can be applied to the shaft.

The advantages of the guard mechanism according to the invention are evident.

In fact such a guard mechanism, comprising only three additional elements, that is to say the sleeve, the spring means, and the spring abutment means, proves extremely cheap and simple to make, and can be applied in a simple and convenient manner to a conventional syringe either automatically or manually to make it into a disposable automatic safety syringe.

The disposable automatic safety syringe obtained with a guard mechanism according to the invention is practical for the user and meets the objects of the present invention.

In fact, once the injection has been completed, the sleeve may automatically and without any intervention by the user move telescopically on the syringe body, so as to cover the needle. In this manner the needle comes to be in a position of safety, thus avoiding accidental needle sticks and possible attempts to reuse the needle, which remains trapped in the sleeve.

Further characteristics of the invention will be made clearer by the detailed description that follows, referring to a purely exemplary and therefore non limiting embodiment thereof, illustrated in the appended drawings, wherein:
Figure 1 is an exploded axonometric view, illustrating the guard mechanism according to the invention and a conventional syringe;
Figure 2 is an axial sectional view of a sleeve forming part of the guard mechanism of Figure 1;
Figure 3 is an axial sectional view of a spring abutment member forming part of the guard mechanism of Figure 1;
Figure 4 is a view, partially in axial section, of the syringe of Figure 1 assembled with the guard mechanism, wherein the shaft of the plunger has not been sectioned and has a safety tab and the needle is shown partially broken off;
Figure 5 is an axial sectional view, like Figure 4, of the syringe assembled with the guard mechanism, wherein the safety tab of the plunger shaft has been removed and the plunger is at its forward end-of-stroke point;
Figure 6 is an axial sectional view, like Figure 4, of the syringe assembled with the guard mechanism, wherein the needle is in the safety position protected by the sleeve and wherein the syringe body and plunger shaft assembly is shown partially broken off;
Figure 7 is an axonometric view of the syringe assembled with the guard mechanism, during use; and
Figure 8 is an axonometric view of the syringe assembled with the guard mechanism in the safety position.

The guard mechanism according to the invention, attachable to a syringe to make it into a disposable automatic safety syringe, is described with the aid of the figures.

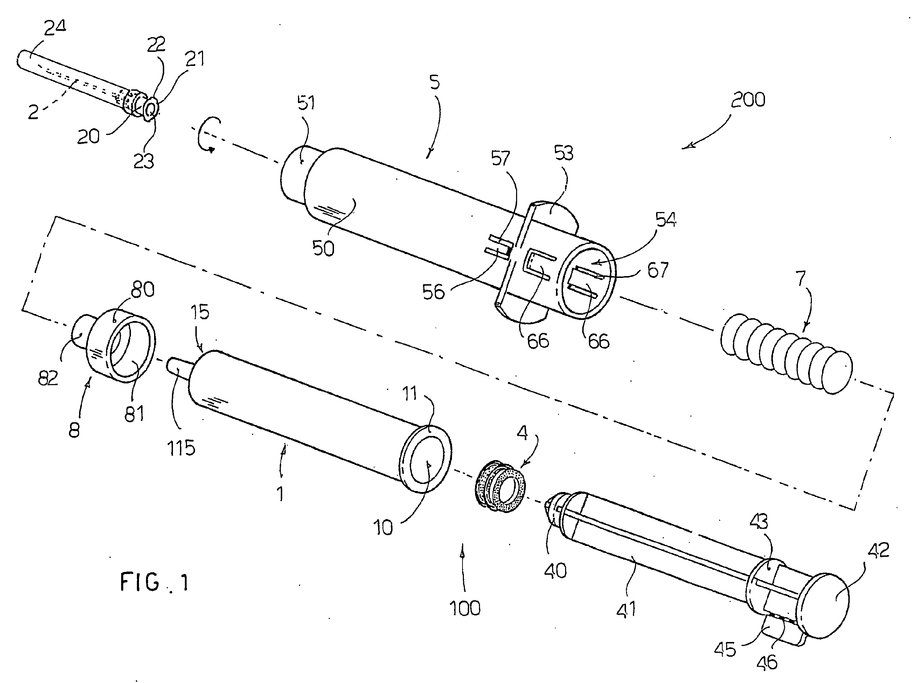

With reference for now in particular to Figure 1, there is illustrated a conventional syringe, denoted as a whole with reference numeral 100, and guard mechanism consisting of a set of components, denoted as a whole by reference numeral 200.

The syringe 100 is a syringe commonly available on the market and comprises a syringe body 1, an injection needle 2, a plunger 4 and a shaft 41.

The syringe body 1 is cylindrical, hollow on the inside, and defines a cylindrical chamber 10. The rear end of the body 1 is outwardly open and has an annular collar 11 that protrudes radially outward. Two tongues or flanges (not shown) that protrude radially outward, in diametrically opposite positions, can be provided on the annular rim 11 to define gripping means for the user's fingers.

The front end of the body 1 ends in a head 115, outwardly open, having the shape of a substantially cylindrical or frusto-conical tang, with a much smaller diameter than the body 1, so as to define a shoulder 15 on the front part of the syringe body 1.

The injection needle 2 is mounted on or built-in in a cylindrical or frusto-conical needle-carrier 20, hollow on the inside, having an axial chamber 23 able to receive the tang 115 of the head of the syringe body. The needle-carrier 20 can have, at its free end, a collar 21 with two tongues 22 protruding radially in diametrically opposite positions. The tongues 22 can be replaced by an outer thread. A needle cap 24 engages with the needle-carrier 20 to cover the needle 2.

The plunger 4 can slide sealingly inside the chamber 10 of the syringe body 1. The plunger 4 is mounted on the head 40 of the shaft 41. The shaft 41 is cross-shaped in cross section and ends at the rear in a disc-shaped flange 42 which provides an abutment surface for the user's fingers during the injection.

Near the rear flange 42, around the shaft 41, an operating crown or disc 43 can be provided. A safety tongue 45 that protrudes radially and longitudinally outward from the shaft 41 is disposed between the rear flange 42 and the operating crown 43. The safety tab 45 is connected to the shaft 41 by means of a breakable connecting strip 46, such as a strip of perforated material acting as a line of weakening. In this manner the user can remove the safety tongue 45 manually by tearing the line of weakening 46.

The operating crown 43 can be made in a single body with the shaft 41 or with the rear flange 42 of the shaft 41. In the case of the shaft 41 of the conventional syringe 100 not having the operating crown 43, said operating crown 43 can be made as a separate element forming part of the set of components 200 and thus able to be assembled to the shaft 41, as shown in the figures.

The set of components 200 comprises a sleeve 5, a spring 7, an abutment member 8 for the spring and optionally an operating crown 43.

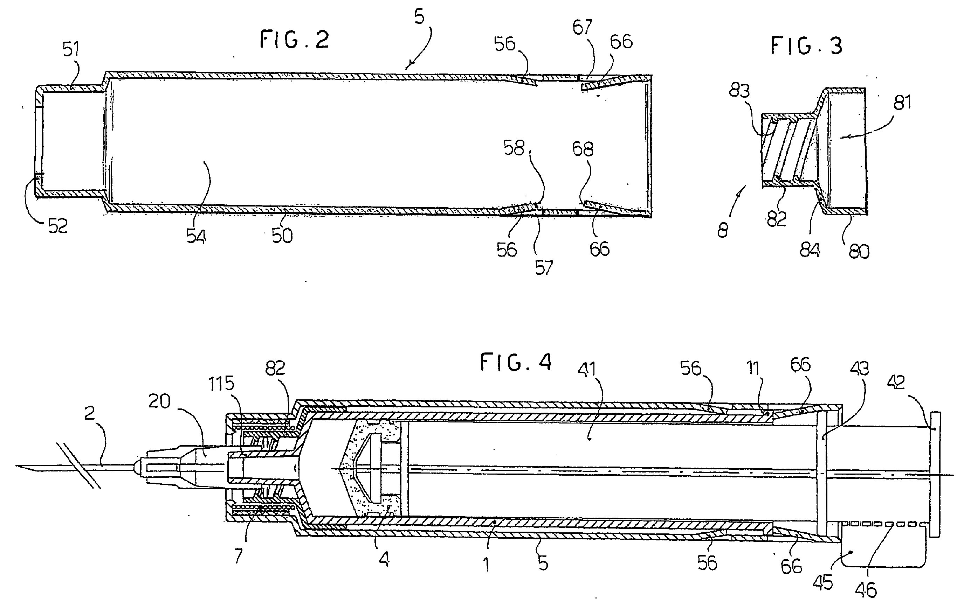

As shown also in Figure 3, the abutment member 8 has a cylindrical or frusto-conical body 80 hollow on the inside, having an axial cavity 81 with a diameter substantially equal to the outside diameter of the front part of the syringe body 1 to be able to attached thereto by pressure. The abutment member 8 has at the front and axially a cylindrical or frusto-conical tang 82, with a smaller diameter than that of the body 80, so as to give rise to a shoulder 84. The tang 82 is hollow on the inside and has an inside thread 83.

In this manner, when the abutment member 8 is applied to the head of the syringe body, the tang 115 of the syringe body is disposed axially inside the tang 82 of the abutment member, leaving an annular space between the outer surface of the tang 115 of the syringe body and the inner surface of the tang 82 of the needle-carrier member, so as to give rise to a so-called Luer cone on the head of the syringe body to receive the needle-carrier 20 of the injection needle 2. In this case, the abutment member 8 acts as a supporting member for the needle 2.

As shown also in Figure 2, according to the invention, the set of components 200 comprises a safety device for the syringe, denoted by reference numeral 5 and taking the form of a substantially cylindrical sleeve, hollow on the inside, having an axial chamber open at the front and rear. The sleeve 5 has a front part 51 with a smaller diameter ending in an annular collar 52 that protrudes radially inward. The inside diameter of the body 50 of the sleeve body is slightly greater than the outside diameter of the body 80 of the abutment member 8, so that the sleeve 5 can slide axially on the body 80 of the abutment member 8, when the abutment member 8 is applied to the head of the syringe body 1.

Near the rear part of the body 50 of the sleeve there are provided two rigid tongues or flanges 53 which protrude radially outward, in diametrically opposite directions, to give rise to a resting surface for the user's fingers.

Two pairs of longitudinal flexible tongues 56, 66 are provided in the rear part of the body 50 of the sleeve, forward and rearward with respect to the flanges 53, respectively.

The first pair of tongues 56 is disposed forward of the flanges 53 and has two tongues 56 disposed in diametrically opposite positions. Each front tongue 56 is slightly inclined towards the inside and ends in a rear abutment surface 58. Each front tongue 56 is defined by a substantially U-shaped cut 57 made on the body of the sleeve, so as to be able to bend with respect to the sleeve body.

The second pair of tongues 66 is disposed to the rear of the flanges 53 and has two tongues 66 disposed in diametrically opposite positions. Each rear tongue 66 is inclined slightly inward and ends in a front abutment surface 68, substantially opposed to the rear abutment surface 58 of the respective front tongue 56. Each rear tongue 66 is defined by a substantially U-shaped cut 67 made in the body of the sleeve, so as to be able to bend with respect to the body of the sleeve.

Lastly, the set of components 200 comprises the spiral spring 7, designed to be housed in the front part 52 of the sleeve body. In fact the outside diameter of the spring 7 is slightly smaller than the inside diameter of the front part 51 of the sleeve body 5.

Assembly of the set of components 200 on the syringe 100 will be described hereunder, purely by way of example, with the aid also of Figure 4.

The guard mechanism 200 can be supplied pre-assembled. That is to say, the spring 7 is inserted in the sleeve 5, from the rear of the sleeve 5, so that the end of the spring abuts against the collar 52 of the front part of the sleeve 5. The abutment member 8 for the spring is then inserted into the sleeve, again from the rear of the sleeve, so that the shoulder 84 of the abutment member abuts against the other end of the spring. Then, by lightly forcing the abutment member 8 into the sleeve, the body 80 of the abutment member passes the pair of rear tongues 66 and thus the rear edge of the abutment member is blocked by the front abutment surface 68 of the rear tongues of the sleeve, preventing the abutment member from coming out of the sleeve.

At this point the pre-assembled protection mechanism 200 can be attached to a conventional syringe 100, mechanically or manually. In fact, it is sufficient to force the syringe body 1 into the sleeve 5, from the rear of the sleeve. In this manner the tang 115 of the syringe body is inserted into the abutment member 8 until the shoulder 15 of the syringe body 1 abuts against the shoulder 84 of the abutment member. In this situation the abutment member 8 is integral with the front part of the syringe body 1 and a Luer cone consisting of the inside tang 115 of the syringe body and the outside tang 82 of the abutment member is formed in the head of the syringe body. By advancing the syringe body and causing the tongues 56 to bend slightly outwards, possibly with the aid of suitable means, the syringe body pushes the abutment member 8 slightly forward, compressing the spring in the front part 51 of the sleeve.

As shown in Figure 4, when the rear edge 11 of the syringe body passes the pair of rear tongues 66 of the sleeve, the front abutment surface 68 of the rear tongues 66 abuts against the rear edge 11 of the syringe body, preventing the syringe body from coming out of the sleeve 5 through the action of the spring 7. At this point the syringe 100 with the guard mechanism 200 is ready for use.

It should be noted that in this situation the safety tab 45 fixed to the shaft 41 abuts against the rear edge of the sleeve 5, preventing the shaft 41 from advancing further, so that the operating crown 43 does not cooperate with the rear tongues 66 of the sleeve, triggering the guard mechanism 200.

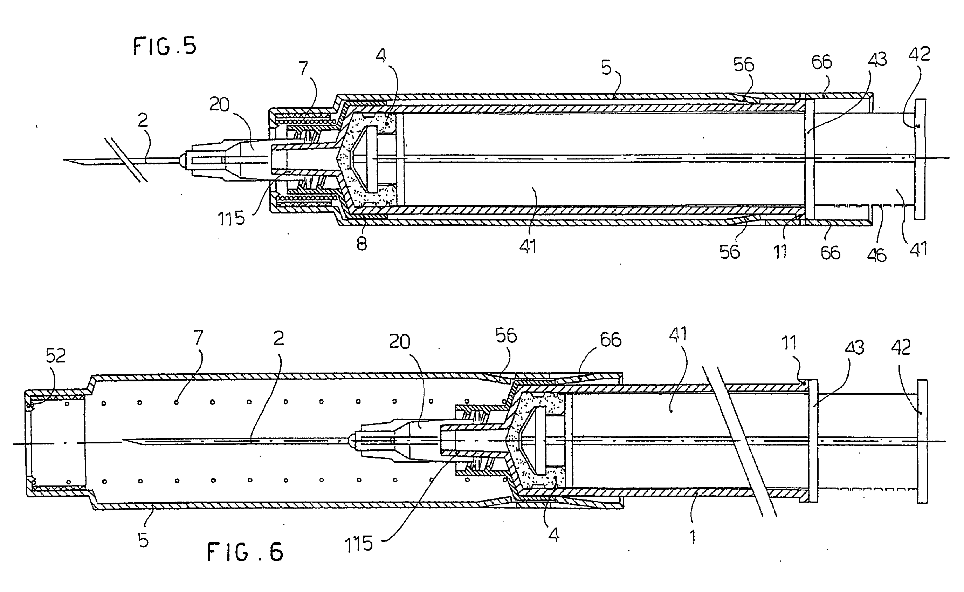

Operation of the syringe 100 provided with the guard mechanism 200 according to the invention is described hereunder with reference to Figures 5 to 8.

Before carrying out the injection, the user manually tears the safety tab 45 along the perforated line of weakening 46.

As shown in Figure 5, when the plunger 4 reaches the end of its injection stroke inside the chamber of the syringe body, the operating crown 43 of the shaft 41 comes into contact with the flexible rear tongues 66 of the sleeve, causing outward bending of said tongues 66.

As a result, the rear edge 11 of the syringe body disengages from the protrusions 68 of the rear tongues 66. Consequently, axial movement of the sleeve 5 with respect to the syringe body 1 is no longer prevented. Thus through the action of the spring 7 which is released, the sleeve 5 can move axially forward with respect to the syringe body 1 and/or the syringe body 1 can move axially rearward with respect to the sleeve 5. It should be noted movement of the sleeve 5 takes place automatically through the action of the spring 7 without the need for any manual intervention by the user.

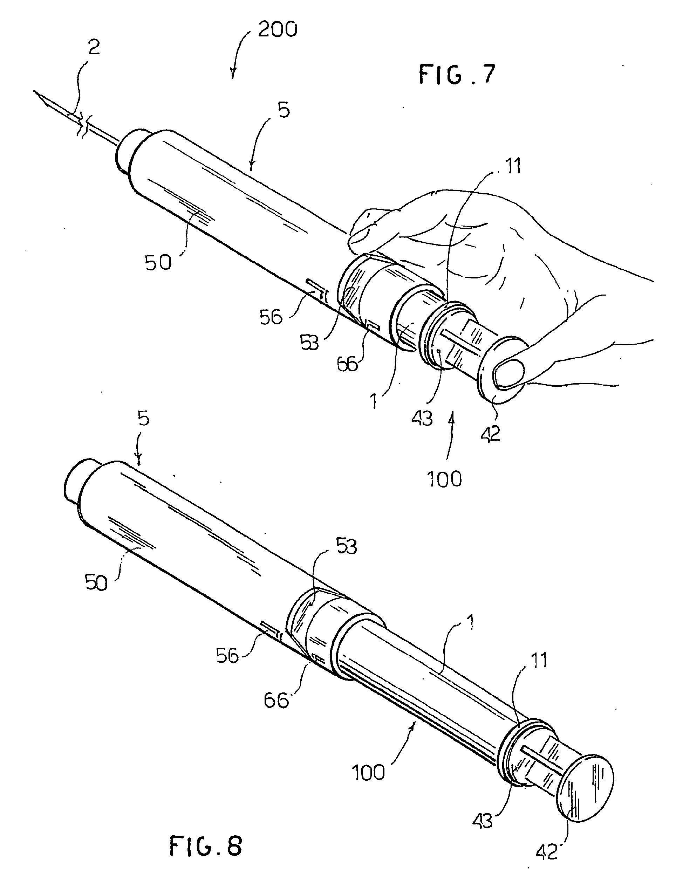

As shown in Figure 7, said telescopic movement of the sleeve 5 and of the syringe 100 is controlled and regulated by the user's hand. In fact the user keeps the index and middle finger on the flanges 53 of the sleeve and the thumb on the rear flange 42 of the plunger shaft, accompanying the stroke of the sleeve 5 and of the syringe 100 towards the position of safety and thus regulating the speed of relative movement of the sleeve 5 with respect to the syringe 1 as desired by the user.

At the end of the stroke of the sleeve 5, as shown in Figures 6 and 8, the body 80 of the abutment member 8 is retained between the two pairs of tongues 56 and 66. That is to say, the rear abutment surface 58 of the front tongues 56 abuts against the shoulder 84 of the abutment member, preventing forward movement thereof, whilst the front abutment surface 68 of the rear tongues 66 abuts against the rear edge of the body 80 of the abutment member, preventing rearward movement thereof. Detachment of the abutment member 8 from the syringe body can be caused by exerting a strong forward traction on the sleeve, but the needle 2 nevertheless remains trapped, together with the abutment member, in the sleeve 5.

It should be noted that in the present invention, the spring 7 remains protected inside the sleeve 5, even when the sleeve 5 is in its forward safety position.

It should further be noted that in the present invention, a syringe of a conventional type, composed of the body 1, the plunger 4 with the shaft 41 thereof and the needle 2 with the support 20 thereof, is made into a safety syringe by the use of only three additional elements forming part of the guard mechanism 200, that is to say, the sleeve 5, the spring 7 and the abutment member 8. Furthermore, the set of components 200 can optionally also comprise the operating crown 43 to be applied to the shaft 41 of the piston, in the event of said operating crown 43 not being made integral or in a single body with the shaft 41.

Numerous variations or modifications of detail within the reach of a person skilled in the art can be made to the present embodiment of the invention, without thereby departing from the scope of the invention as set forth in the appended claims.

## Claims

1. A guard mechanism (200) attachable to a syringe (100) with an injection needle to make it into a disposable automatic safety syringe,
said guard mechanism (200) being pre-assemblable and comprising:
- a sleeve (5) that can be slidably mounted on a syringe body (1) and adapted, to pass from a retracted position of use of the syringe, to a forward position of safety wherein it can cover said needle,
- a spring (7) housed in said sleeve (5),
- an abutment member (8) for said spring (7), also housed in said sleeve (5) and able to be applied to the front part of said syringe body (1),
**characterised in that**
said abutment member (8) comprises:
- a cylindrical or frusto-conical body (80) having a rear edge, the body being hollow on the inside to be applied to the front part of the syringe body (1), and
- a cylindrical or frusto-conical tang (82) with a smaller diameter than the body (80) and protruding forward therefrom so as to give rise to a shoulder (84) and
said sleeve (5) comprises a pair of front tongues (56) opposed to a pair of rear tongues (66), said pair of front tongues (56) having rear abutment surfaces (58) able to abut against the shoulder (84) of the abutment member (8) and said pair of rear tongues (66) having front abutment surfaces (68) able to abut against the rear edge of the body (80) of the abutment member, in order to lock the sleeve (5) when it is in said forward position of safety.

2. A mechanism (200) according to claim 1, **characterised in that** in said pre-assembled condition, said abutment member (8) for said spring (7) is retained by said rear tongues (66) in the form of flexible tongues protruding inward from said sleeve (5).

3. A mechanism (200) according to claim 1 or 2, **characterised in that** said spring (7) is disposed under compression in the front part of said sleeve (5), between said sleeve (5) and said abutment member (8) made integral with said syringe body (1), to urge the axial movement of the sleeve (5) with respect to the syringe body,
- said rear tongues (66) of the sleeve being engageable with a collar (11) disposed in the rear part of the syringe body (1), to keep the sleeve locked in the retracted position of use against the action of said spring (7), and
- said rear tongues (56) of the sleeve being releasable by operating means (43) disposed in a shaft (41) of the syringe, when the plunger (4) of the syringe reaches the end of the injection stroke, so as to allow the axial movement of the sleeve into the safety position, thanks to the action of said spring (7).

4. A mechanism according to any one of the preceding claims, **characterised in that** said spring is a spiral spring (7) disposed in the front part (51) of the sleeve, around the tang (82) of the abutment member, with one end of the spring (7) abutting against a collar (52) protruding inward in the front edge of the front part (51) of the sleeve and the other end of the spring abutting against the shoulder (84) of the abutment member.

5. A mechanism (200) according to any one of the preceding claims, **characterised in that** said pairs of opposed tongues (56, 66) of the sleeve are flexible and are formed by means of substantially U-shaped opposed cuts (57, 67) in the sleeve body, to be able to bend radially inward and outward with respect to the sleeve.

6. A mechanism according to claim any one of the preceding claims, **characterised in that** said flexible tongues (66) are inclined slightly inward to cooperate with a circular operating crown (43) of the syringe shaft, when the plunger is at the end of the injection stroke.

7. A mechanism according to any one of the preceding claims, **characterised in that** said sleeve (5) has outwardly protruding gripping means (53), to give rise to a resting surface for the user's fingers.

8. A syringe (100) provided with a guard mechanism (200) according to any one of the preceding claims.

## Patentansprüche

1. Schutzmechanismus (200), der an einer Spritze (100) mit einer Injektionsnadel angebracht werden kann, um sie zu einer wegwerfbaren automatischen Sicherheitsspritze zu machen, wobei der Schutzmechanismus (200) vormontierbar ist und Folgendes umfasst:
- eine Hülse (5), die gleitfähig an einem Spritzenkörper (1) montiert werden kann und die dafür geeignet ist, von einer eingeschobenen Gebrauchsposition der Spritze bis zu einer vorgeschobenen Sicherheitsposition zu reichen, in der sie die Nadel abdecken kann,
- eine Feder (7), die in der Hülse (5) angeordnet ist,
- ein Widerlagerelement (8) für die Feder (7), das ebenfalls in der Hülse (5) untergebracht ist und am vorderen Teil des Spritzenkörper (1) angebracht werden kann,
**dadurch gekennzeichnet, dass**
das Widerlagerelement (8) Folgendes umfasst:
- einen zylindrischen oder kegelstumpfförmigen Körper (80), der eine Hinterkante aufweist, wobei der Körper innen hohl ist, um an den vorderen Teil des Spritzenkörpers (1) angesetzt zu werden, und
- einen zylindrischen oder kegelstumpfförmigen Ansatz (82), der einen kleineren Durchmesser als der Körper (80) aufweist und der von dem Körper (80) nach vom hervorsteht, so dass eine Schulter (84) entsteht, und wobei die Hülse (5) ein Paar vorderer Zungen (56) umfasst, die einem Paar hinterer Zungen (66) gegenüberliegen, wobei das Paar vorderer Zungen (56) hintere Anschlagflächen (58) aufweist, die an der Schulter (84) des Widerlagerelements (8) anliegen können, und das Paar hinterer Zungen (66) vordere Anschlagflächen (68) aufweist, die an der Hinterkante des Körper (80) des Widerlagerelement anliegen können, um die Hülse (5) zu verriegeln, wenn sie sich in der vorgeschobenen Sicherheitsposition befindet.

2. Mechanismus (200) nach Anspruch 1, **dadurch gekennzeichnet, dass** in dem vormontierten Zustand das Widerlagerelement (8) für die Feder (7) durch die hinteren Zungen (66) in der Form flexibler Zungen, die von der Hülse (5) nach innen ragen, gehalten wird.

3. Mechanismus (200) nach Anspruch 1 oder 2, **dadurch gekennzeichnet, dass:**
- die Feder (7) unter Druck in dem vorderen Teil der Hülse (5) zwischen der Hülse (5) und dem Widerlagerelement (8), das integral mit dem Spritzenkörper (1) zusammengesetzt ist, angeordnet ist, um die axiale Bewegung der Hülse (5) relativ zu dem Spritzenkörper zu bewirken,
- die hinteren Zungen (66) der Hülse mit einem Bund (11) in Eingriff gebracht werden können, der im hinteren Teil des Spritzenkörpers (1) angeordnet ist, um die Hülse in der eingeschobenen Gebrauchsposition entgegen der Wirkung der Feder (7) arretiert zu halten, und
- die hinteren Zungen (56) der Hülse mit Hilfe von Betätigungsmitteln (43), die in einem Schaft (41) der Spritze angeordnet sind, freigegeben werden können, wenn der Stempel (4) der Spritze das Ende des Injektionshubes erreicht, um die axiale Bewegung der Hülse dank der Wirkung der Feder (7) in die Sicherheitsposition zu gestatten.

4. Mechanismus nach einem der vorangehenden Ansprüche, **dadurch gekennzeichnet, dass** die Feder eine Spiralfeder (7) ist, die in dem vorderen Teil (51) der Hülse um den Ansatz (82) des Widerlagerelements herum angeordnet ist, wobei ein Ende der Feder (7) an einem Bund (52) anliegt, der im vorderen Rand des vorderen Teils (51) der Hülse nach innen hervorsteht, und wobei das andere Ende der Feder an der Schulter (84) des Widerlagerelements anliegt.

5. Mechanismus (200) nach einem der vorangehenden Ansprüche, **dadurch gekennzeichnet, dass** die Paare gegenüberliegender Zungen (56, 66) der Hülse flexibel sind und mittels im Wesentlichen U-förmiger gegenüberliegender Einschnitte (57, 67) in dem Hülsenkörper gebildet sind, um sich mit Bezug auf die Hülse radial nach innen und außen zu biegen.

6. Mechanismus nach einem der vorangehenden Ansprüche, **dadurch gekennzeichnet, dass** die flexiblen Zungen (66) leicht nach innen geneigt sind, um mit einer kreisförmigen Betätigungskrone (43) des Spritzenschaftes zusammenzuwirken, wenn sich der Stempel am Ende des Injektionshubes befindet.

7. Mechanismus nach einem der vorangehenden Ansprüche, **dadurch gekennzeichnet, dass** die Hülse (5) nach außen hervorstehende Greifmittel (53) aufweist, um eine Auflagefläche für die Finger des Benutzers zu bilden.

8. Spritze (100), die mit einem Schutzmechanismus (200) nach einem der vorangehenden Ansprüche ausgestattet ist.

## Revendications

1. Mécanisme de protection (200) pouvant être fixé à une seringue (100) afin de la transformer en une seringue de sûreté automatique jetable, ledit mécanisme de protection (200) étant pré-assemblable et comprenant :
- un manchon (5) qui peut être monté de manière coulissante sur un corps de seringue (1), et adapté afin de passer d'une position d'utilisation rétractée de la seringue, en une position de sûreté avancée, dans laquelle il peut couvrir ladite aiguille,
- un ressort (7) logé dans ledit manchon (5),
- un élément de butée (8) destiné audit ressort (7), également logé dans ledit manchon (5) et apte à être appliqué sur la partie frontale dudit corps de seringue (1),
**caractérisé en ce que**
ledit élément de butée (8) comprend :
- un corps cylindrique ou frusto-conique (80) ayant un bord arrière, le corps étant creux sur l'intérieur à appliquer sur la partie frontale du corps de seringue (1), et
- un tenon cylindrique ou frusto-conique (82) avec un diamètre plus petit que le corps (80) et dépassant à l'avant de celui-ci de manière à ménager un épaulement (84) et ledit manchon (5) comprend une paire de languettes frontales (56) opposées à une paire de languettes arrières (66), ladite paire de languettes frontales (56) ayant des surfaces de butée arrière (58) aptes à buter contre l'épaulement (84) de l'élément de butée (8) et ladite paire de languettes arrières (66) ayant des surfaces de butée frontales (68) aptes à buter contre le bord arrière du corps (80) de l'élément de butée, de manière à verrouiller le manchon (5) quand il est dans ladite position avancée de sûreté.

2. Mécanisme (200) selon la revendication 1, **caractérisé en ce que** dans ladite condition pré-assemblée, ledit élément de butée (8) destiné audit ressort (7) est retenu par lesdites languettes arrières (66) sous la forme de languettes flexibles dépassant vers l'intérieur à partir dudit manchon (5).

3. Mécanisme (200) selon la revendication 1 ou 2, **caractérisé en ce que** ledit ressort (7) est disposé sous l'effet d'une compression dans la partie frontale dudit manchon (5), entre ledit manchon (5) et ledit élément de butée (8) étant partie intégrante dudit corps de seringue (1), afin de presser le mouvement axial du manchon (5) par rapport au corps de seringue,
- lesdites languettes arrières (66) du manchon étant mises en prise avec un collier (11) disposé dans la partie arrière du corps de seringue (1), afin de garder le manchon verrouillé dans la position d'utilisation rétractée contre l'action dudit ressort (7), et
- lesdites languettes arrières (56) du manchon étant libérables par des moyes opérationnels (43) disposé dans un dit axe (41) de la seringue, lorsque le poussoir (4) de la seringue atteint la fin de la course d'injection, de manière à permettre le mouvement axial du manchon dans la position de sûreté, grâce à l'action dudit ressort (7).

4. Mécanisme selon une quelconque des revendications précédentes, **caractérisé en ce que** ledit ressort est un ressort en spirale (7) disposé dans la partie frontale (51) du manchon, autour du tenon (82) de l'élément de butée, avec une extrémité du ressort (7) butant contre un collier (52) dépassant vers l'intérieur dans le bord frontal de la partie frontale (51) du manchon et l'autre extrémité du ressort butant contre l'épaulement (84) de l'élément de butée.

5. Mécanisme (200) selon une quelconque des revendications précédentes, **caractérisé en ce que** lesdites paires de languettes opposées (56,66) du manchon sont flexibles et sont formées au moyen de découpes (57,67) opposées essentiellement en forme de U dans le corps de manchon, afin d'être aptes à être incurvées radialement vers l'intérieur et l'extérieur par rapport au manchon.

6. Mécanisme selon une quelconque des revendications précédentes, **caractérisé en ce que** lesdites languettes flexibles (66) sont inclinées légèrement vers l'intérieur afin de coopérer avec une couronne (43) opérationnelle circulaire de l'axe de seringue, lorsque le poussoir est à la fin de la course d'injection.

7. Mécanisme selon une quelconque des revendications précédentes, **caractérisé en ce que** ledit manchon (5) possède des moyens de préhension (53) dépassant vers l'extérieur, afin de ménager une surface de repos pour les doigts de l'utilisateur.

8. Seringue (100) pourvu d'un mécanisme de protection (200) selon une quelconque des revendications précédentes.
